# EUROPEAN PATENT SPECIFICATION

(11) **EP 3 315 128 B1**
(45) Date of publication and mention of the grant of the patent: **19.05.2021**
(21) Application number: 15896367.8
(22) Date of filing: 26.06.2015
(51) Int. Cl.: A61K 31/427, A61P 25/00

(54) **DOSING REGIMEN OF ROVATIRELIN FOR ATAXIA ASSOCIATED WITH SPINOCEREBELLAR DEGENERATION**
DOSIERSCHEMA FÜR ROVATIRELIN FÜR MIT ATAXIE EINHERGEHENDE SPINOZEREBELLÄRE DEGENERATION
RÉGIME DE DOSAGE DU ROVATIRELIN CONTRE L'ATAXIE ASSOCIÉE À LA DÉGÉNÉRESCENCE SPINO-CÉRÉBELLEUSE

(43) Date of publication of application: 02.05.2018
(73) Proprietor: Shionogi & Co., Ltd., Osaka 541-0045 (JP)
(72) Inventor: SHIMIZU, Yoshitaka, Tokyo 112-0002 (JP); YAMANO, Hitoshi, Tokyo 112-0002 (JP); KIYONO, Yuji, Tokyo 112-0002 (JP); IJIRO, Tomoyuki, Azumino-shi Nagano 399-8304 (JP)
(74) Representative: Hoffmann Eitle
(86) International application number: PCT/JP2015/068438
(87) International publication number: WO 2016/208045

(56) References cited:
- WO-A1-2006/028277
- JP-A- 2008 512 344
- FROESTL WOLFGANG ET AL: "Cognitive Enhancers (Nootropics). Part 3: Drugs Interacting with Targets other than Receptors or Enzymes. Disease-modifying Drugs", JOURNAL OF ALZHEIMER'S DISEASE, IOS PRESS, NL, vol. 34, no. 1, 1 January 2013 (2013-01-01), pages 1-114, XP009186604, ISSN: 1387-2877, DOI: 10.3233/JAD-121729
- HAJIME KAINUMA ET AL.: 'Clinical Phase I Study of TA-0910. Oral Repeated Dose Study.' JOURNAL OF CLINICAL THERAPEUTICS & MEDICINES vol. 13, no. 10, June 1997, pages 2517 - 2532, XP009504662

## Description

### Technical Field

The present invention relates to pharmaceutical agents with which the risk of side effects caused by elevation of thyroid hormone levels is reduced and exhibit excellent effects in improving ataxia in spinocerebellar degeneration when administered according to particular dosage and administration.

More specifically, the present invention relates to pharmaceutical compositions for use in the treatment of ataxia in spinocerebellar degeneration including, as an active ingredient, a daily dose of 1.6 mg to 3.2 mg of rovatirelin or 1.6 mg to 3.2 mg of pharmacologically acceptable salt of rovatirelin as being calculated as a free form, wherein the pharmaceutical composition is administered once daily.

### Background Art

Spinocerebellar degeneration (SCD) is one of the neurodegenerative diseases that has lesions primarily in cerebellum, spinal cord nuclei and/or conduction pathways and is mainly characterized by progressive cerebellar ataxia. SCD is a disease that occurs at a wide range of ages from young to old. For example, in Japan, SCD is designated as an incurable disease in the nervous system and muscles, and it is estimated that there are approximately 38,000 SCD patients (including those with multiple system atrophy). SCD includes various types, which are generally classified into sporadic and hereditary. Sporadic SCD includes multiple system atrophy (MSA). From the clinical and pathological perspectives, both sporadic and hereditary SCD include two types: one caused by atrophy of the cerebellum alone, which exhibit symptoms of cerebellar ataxia (pure cerebellar SCD), and the other accompanied by atrophy of the brainstem and spinal cord, which exhibit extrapyramidal symptoms and peripheral nerve symptoms in addition to the symptoms of cerebellar ataxia (non-pure cerebellar SCD).

Thyrotropin-releasing hormone (TRH) agent is known as a therapeutic agent for ataxia in SCD. Since the TRH agent has a short duration of action *in vivo* and is made as an injection formulation, its administration requires frequent outpatient visits or inpatient stays. Furthermore, TRH is known to have central actions such as spontaneous hyperactivity and excitation of spinal motor neurons and also have hormonal action that promotes the secretion of thyroid stimulating hormone (TSH) and prolactin (PRL) from the pituitary gland. Therefore, when using a TRH agent, side effects caused by the hormonal action of TRH should be especially noted.

Taltirelin hydrate, a TRH analogue, is also known as a therapeutic agent for ataxia in SCD. Taltirelin hydrate is an orally administrable drug and is known to have a longer duration of action than TRH agents (NPL 1). In addition, the safety and pharmacokinetics of taltirelin hydrate during its continuous oral administration has been reported (NPL 2). In this document, reported was an influence of taltirelin hydrate on hormone values (TSH values, T₃ values, and T₄ values) and the like when it was administered at different doses-2.5 mg twice daily and 5 mg once daily. In a phase III double-blind comparative study, observed was a significant difference in the total improvement level according to a subjective assessment of a physician after 28 weeks between taltirelin hydrate and placebo treatment for ataxia in SCD; however, no significant improvement was observed for individual symptoms (symptom of ataxia) (NPL 3) .

The effect in improving ataxia in SCD can be assessed by the Scale for the Assessment and Rating of Ataxia (SARA). The SARA is a scale published in 2006 for the assessment of ataxia, and is acknowledged to be a valid and reliable scale for the assessment of ataxia. No pharmaceutical agent, however, was found to have an effect in improving ataxia in SCD in clinical trials that used SARA.

Therefore, a novel pharmaceutical agent with which the risk of side effects caused by elevation of thyroid hormone levels was reduced, and which was far more effective in improving ataxia in SCD, was desired.

Azetirelin, DN-1417, JTP-2942, MK-771, montirelin, posatirelin, and RX-77368, all of which are TRH analogues, were evaluated in clinical trials for indications such as the improvement of symptoms of a cerebrovascular disorder, improvement of a persistent vegetative state, and the treatment of Alzheimer's disease. All of these clinical developments were, however, discontinued. Although TRH analogues attracted attention for their TRH-like actions and clinical developments were performed for various indications, most clinical developments of TRH analogues were extremely challenging. Accordingly, the quest for finding the dosage and administration of TRH analogues which could be both effective and safe was considered challenging.

(4S,5S)-5-methyl-N-{(2S)-1-[(2R)-2-methylpyrrolidin-1-yl]-1-oxo-3-(1,3-thiazol-4-yl)propan-2-yl}-2-oxo-1,3-oxazolidine-4-carboxamide (generic name: rovatirelin) represented by the formula (I) is described as a TRH analogue (PTL 1). In addition, rovatirelin is known as a therapeutic agent for SCD (PTL 2). PTL 2 also describes that rovatirelin has a high bioavailability (BA) and exhibited excellent effects in improving ataxia, which was at least 30 times higher than those of taltirelin in animal models. It is, however, anticipated that such an increase in efficacy and BA would also lead to the enhancement of hormonal effects of the TRH analogue. Therefore, it is not easy to establish dosage and administration of rovatirelin for reducing the risk of side effects caused by elevation of thyroid hormone levels and exhibiting excellent effects in improving ataxia in spinocerebellar degeneration.

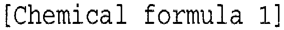

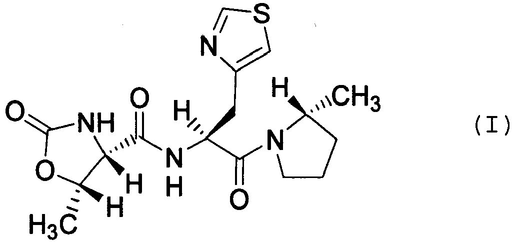

### Citation List

### Patent Literature

PTL 1: WO99/53941
PTL 2: JP2008-512344
PTL 3: WO2006/028277

### Non Patent Literature

NPL 1: Keizo Hirayama, et al., Journal of Clinical Therapeutics & Medicine, 1997, 13(16), p. 4133-4167
NPL 2: Hajime Kainuma et al., Journal of Clinical Therapeutics & Medicine, 1997, 13(10), p. 2517-2532
NPL 3: Ichiro Kanazawa et al., Journal of Clinical Therapeutics & Medicine, 1997, 13(16), p.4169-4224

### Summary of Invention

### Technical Problem

It is an object of the present invention to provide an excellent pharmaceutical composition for the treatment of ataxia in SCD, with which the risk of incidence of side effects caused by elevation of thyroid hormone levels is reduced.

### Solution to Problem

As described above, the dosage and administration of taltirelin hydrate which is the only known therapeutic agent for ataxia in SCD among the TRH analogues have been determined to a twice daily oral dose of 5 mg according to the safety tests, dose response exploratory study and the like, which were described in NPL 2. NPL 2 shows that influences on TSH, T₃, and T₄ are often lower when administered twice daily than when administered once daily. Therefore, it was suggested that it would be better to administer a TRH analogue in multiple divided doses, considering the risk of incidence of side effects caused by elevation of thyroid hormone levels. However, after extensive studies conducted to solve the problems mentioned above, the present inventors unexpectedly found that using rovatirelin for the treatment of ataxia in SCD reduces influences of elevations in thyroid hormone levels when administered once daily than when administered two times or more daily.

Specifically, the present invention relates to the following [1] to [7] and the like.
[1] A pharmaceutical composition for use in the treatment of ataxia in spinocerebellar degeneration including, as an active ingredient, a daily dose of 1.6 mg to 3.2 mg of rovatirelin or 1.6 mg to 3.2 mg of pharmacologically acceptable salt of rovatirelin as being calculated as a free form, wherein the pharmaceutical composition is administered once daily.
[2] The pharmaceutical composition according to [1] for use according to [1], wherein 1.6 mg to 3.2 mg of rovatirelin trihydrate as being calculated as a free form is included as the active ingredient.
[3] The pharmaceutical composition according to [1] for use according to [1], wherein a daily dose of 2.4 mg of rovatirelin or 2.4 mg of pharmacologically acceptable salt of rovatirelin as being calculated as a free form is included as the active ingredient.
[4] The pharmaceutical composition according to [3] for use according to [3], wherein 2.4 mg of rovatirelin trihydrate as being calculated as a free form is included as the active ingredient.
[5] The pharmaceutical composition according to any one of [1] to [4] for use according to any one of [1] to [4], wherein the ataxia in spinocerebellar degeneration is cerebellar ataxia in spinocerebellar degeneration.
[6] The pharmaceutical composition according to any one of [1] to [5] for use according to any one of [1] to [5], wherein the pharmaceutical composition is an oral agent including rovatirelin or a pharmacologically acceptable salt thereof, and at least one pharmaceutical additive.
[7] A pharmaceutical composition for use in the treatment of ataxia in spinocerebellar degeneration comprising, as an active ingredient, a daily dose of 1.6 mg, 2.4 mg, or 3.2 mg of rovatirelin or 1.6 mg, 2.4 mg, or 3.2 mg of pharmacologically acceptable salt of rovatirelin as being calculated as a free form in a single formulation.

### Advantageous Effects of Invention

The pharmaceutical compositions used in the present invention have excellent effects in improving ataxia in SCD.

### Brief Description of Drawings

Doses of rovatirelin trihydrate in the brief description of the drawings represent values calculated as a free form unless otherwise specified.

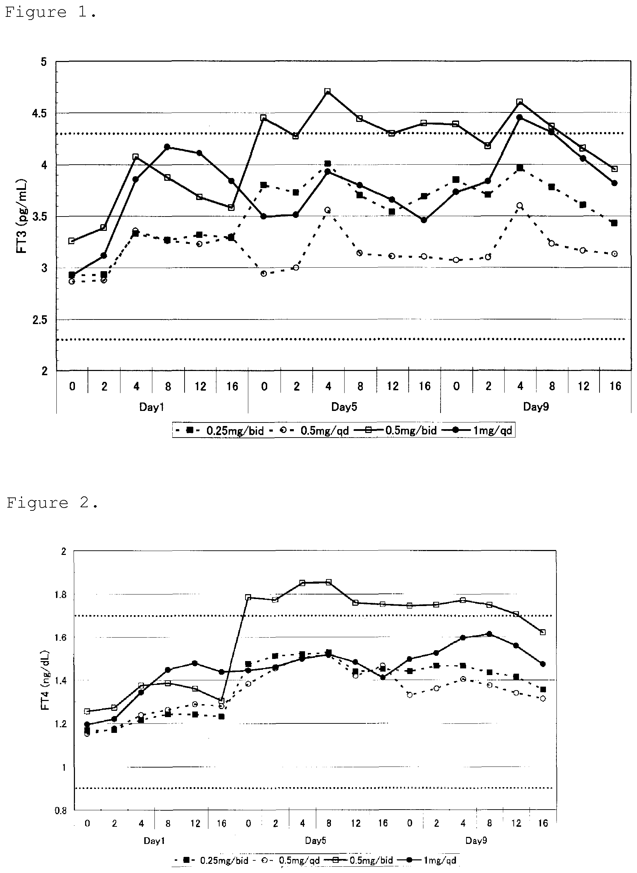

[Fig. 1] Fig. 1 shows the transition of FT₃ serum concentrations in each group (i.e., mean values in 8 patients per group). The horizontal axis represents the time frame of the measurement. "Day 1," "Day 5," and "Day 9" indicate the 1st, 5th, and 9th days, respectively, from the beginning of the administration of the investigational drug. Numerals from 0-16 indicate time in hours from the administration after breakfast. The vertical axis represents FT₃ values (pg/mL). In the figure, solid squares (on a dashed line) represent values for the treatment group that received 0.25 mg twice daily (0.25 mg/bid), open circles (on a dashed line) represent values for the treatment group that received 0.5 mg once daily (0.5 mg/qd), open squares (on a solid line) represent values for the treatment group that received 0.5 mg twice daily (0.5 mg/bid), and solid circles (on a solid line) represent values for the treatment group that received 1 mg once daily (1 mg/qd). Dotted lines represent reference limits (2.3 and 4.3 pg/mL).
[Fig. 2] Fig. 2 shows the transition of FT₄ serum concentrations in each group (i.e., mean values in 8 patients per group). The horizontal axis represents the time frame of the measurement as in Fig. 1. The vertical axis represents FT₄ values (ng/dL). In the figure, solid squares (on a dashed line) represent values for the treatment group that received 0.25 mg twice daily (0.25 mg/bid), open circles (on a dashed line) represent values for the treatment group that received 0.5 mg once daily (0.5 mg/qd), open squares (on a solid line) represent values for the treatment group that received 0.5 mg twice daily (0.5 mg/bid), and solid circles (on a solid line) represent values for the treatment group that received 1 mg once daily (1 mg/qd). Dotted lines represent reference limits (0.9 and 1.7 ng/dL).

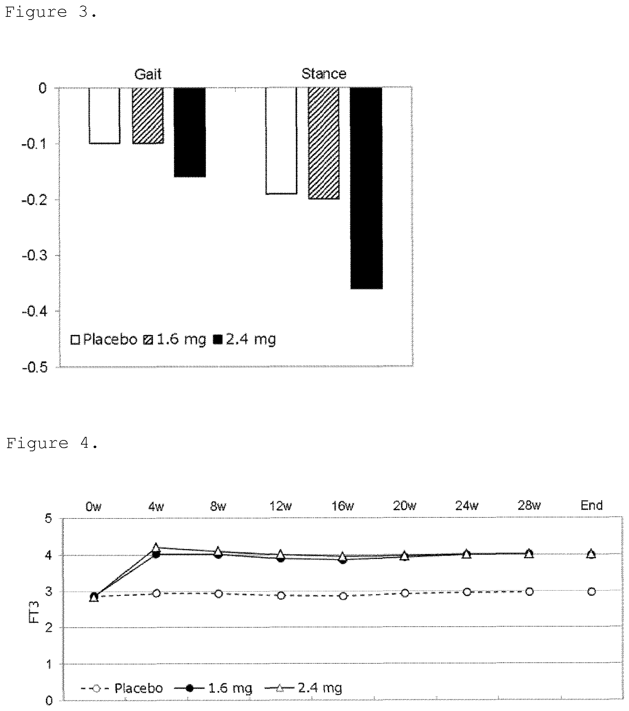

[Fig. 3] Fig. 3 shows the amounts of change in SARA gait scores and SARA stance scores for SCD patients (i.e., mean scores in 122-124 patients per group). The bars in the graph represent, from the left, SARA gait scores (Gait) for the placebo group (Placebo), the treatment group that received 1.6 mg of rovatirelin trihydrate (1.6 mg), and the treatment group that received 2.4 mg of rovatirelin trihydrate (2.4 mg), and SARA stance scores (Stance) for the placebo group (Placebo), the treatment group that received 1.6 mg of rovatirelin trihydrate (1.6 mg), and the treatment group that received 2.4 mg of rovatirelin trihydrate (2.4 mg). The vertical axis represents amount of change in the SARA gait scores or the SARA stance scores.
[Fig. 4] Fig. 4 shows the transition of FT₃ serum concentrations in each group (i.e., mean values in 123-126 patients per group). The horizontal axis represents the time frame of the measurement. "4W" to "28W" represent 4 to 28 weeks from the beginning of the administration of the investigational drug, respectively. "End" represents the time of final assessment. The vertical axis represents FT₃ values (pg/mL). In the figure, solid circles (on a solid line) represent values for the treatment group that received 1.6 mg of rovatirelin trihydrate (1.6 mg), open triangles (on a solid line) represent values for the treatment group that received 2.4 mg of rovatirelin trihydrate (2.4 mg), and open circles (on a dashed line) represent values for the placebo group (Placebo).

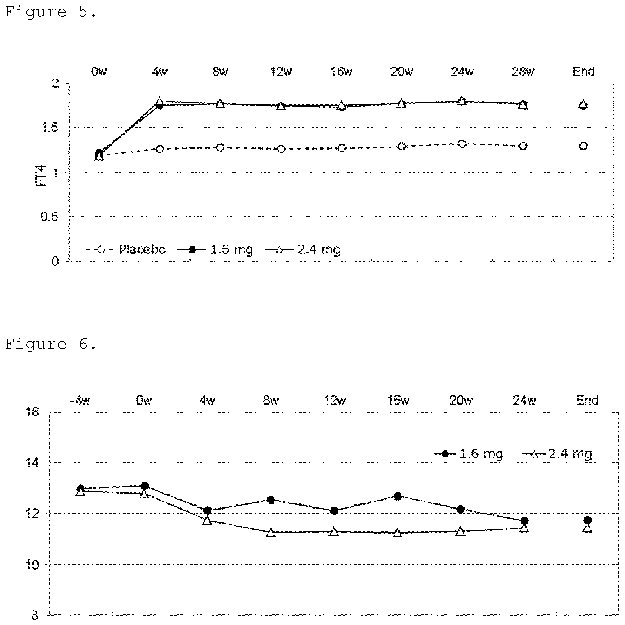

[Fig. 5] Fig. 5 shows the transition of FT₄ serum concentrations in each group (i.e., mean values in 123-126 patients per group). The horizontal axis represents the time frame of the measurement. "4W" to "28W" represent 4 to 28 weeks from the beginning of the administration of the investigational drug, respectively. "End" represents the time of final assessment. The vertical axis represents FT₄ values (ng/dL). In the figure, solid circles (on a solid line) represent values for the treatment group that received 1.6 mg of rovatirelin trihydrate (1.6 mg), open triangles (on a solid line) represent values for the treatment group that received 2.4 mg of rovatirelin trihydrate (2.4 mg), and open circles (on a dashed line) represent values for the placebo group (Placebo).
[Fig. 6] Fig. 6 shows the transition of SARA total scores for SCD patients who switched from taltirelin to rovatirelin trihydrate (i.e., mean scores in 19 or 23 patients per group). The horizontal axis represents the time frame of the measurement. "-4W" represents four weeks prior to the beginning of the administration of the investigational drug. "4W" to "24W" represent 4 to 24 weeks from the beginning of the administration of the investigational drug, respectively. "End" represents the time of final assessment. The vertical axis represents SARA total scores. In the figure, solid circles (on a solid line) represent values for the treatment group that received 1.6 mg of rovatirelin trihydrate (1.6 mg), and open triangles (on a solid line) represent values for the treatment group that received 2.4 mg of rovatirelin trihydrate (2.4 mg).

### Description of Embodiments

Hereinafter, embodiments of the present invention are described in more detail.

In the present invention, each term has the following meaning unless otherwise specified.

The term "rovatirelin" refers to, as described above, the compound represented by the formula (I) ((4S,5S)-5-methyl-N-{(2S)-1-[(2R)-2-methylpyrrolidin-1-yl]-1-oxo-3-(1,3-thiazol-4-yl)propan-2-yl}-2-oxo-1,3-oxazolidine-4-carboxamide). Rovatirelin trihydrate is listed as "rovatirelin hydrate" in Japanese Accepted Names for Pharmaceuticals (JAN).

In the present invention, rovatirelin can be converted to a pharmacologically acceptable salt thereof according to a routine method, if necessary. Examples of such salts include salts of rovatirelin with an alkali metal (e.g., lithium, sodium or potassium), an alkaline earth metal (e.g., magnesium or calcium), ammonium, an organic base, and an amino acid as well as salts of rovatirelin with an inorganic acid (e.g., hydrochloric acid, hydrobromic acid, phosphoric acid, or sulfuric acid), and an organic acid (e.g., acetic acid, citric acid, maleic acid, fumaric acid, benzenesulfonic acid or p-toluenesulfonic acid).

In the present invention, the term "pharmacologically acceptable salt of rovatirelin" also includes solvates of rovatirelin with a pharmaceutically acceptable solvent such as water and ethanol. Among the hydrates included in the pharmacologically acceptable salt of rovatirelin, rovatirelin monohydrate or rovatirelin trihydrate is preferable, and rovatirelin trihydrate is particularly preferable. In the present invention, as used in conjunction with the dose of the pharmacologically acceptable salt of rovatirelin, the term "calculated as a free form" indicates the value as rovatirelin.

Rovatirelin and pharmacologically acceptable salts thereof used in the present invention can be produced using a known method. For example, rovatirelin and rovatirelin trihydrate according to the present invention can be produced by the method described in PTL 3 (WO2006/028277; Published Japanese Translation No. 2008-512344) or a method based on it.

Various dosage forms can be used for the pharmaceutical compositions depending on modes of administration. Examples of such dosage forms include tablets, granules, fine granules, dry syrups, and capsules, which are orally administered.

Each pharmaceutical composition is prepared using rovatirelin or a pharmacologically acceptable salt thereof and at least one pharmaceutical additive. These pharmaceutical compositions may be formulated by appropriately mixing, diluting, or dissolving them with or in a pharmaceutical additive such as an appropriate excipient, disintegrant, binder, lubricant, diluent, buffer, tonicity agent, preservative, wetting agent, emulsifying agent, dispersing agent, stabilizing agent, and solubilizing agent using a method that is known in the pharmacological field, depending on the dosage form of the preparations.

In the present invention, the term "ataxia in SCD" includes cerebellar ataxia in SCD and the like, and does not include secondary ataxia without SCD (e.g., ataxia accompanied by cerebrovascular disorder, brain tumor and the like).

In the present invention, the term "pure cerebellar SCD" refers to types caused by atrophy of the cerebellum alone which exhibit symptoms of cerebellar ataxia. Autosomal dominant pure cerebellar SCD is also referred to as autosomal dominant cerebellar ataxia (ADCA) type III, according to Harding's classification. The pure cerebellar SCD includes SCA6, SCA31 and the like, which are genetic SCD, and also includes sporadic cortical cerebellar atrophy (CCA) and the like.

In the present invention, the term "non-pure cerebellar SCD" refers to types accompanied by atrophy of the brainstem and spinal cord which exhibit symptoms of extrapyramidal symptoms, peripheral nerve symptoms and other symptoms in addition to symptoms of cerebellar ataxia. Autosomal dominant non-pure cerebellar SCD is also referred to as ADCA type I, and may be referred to as ADCA type II or ADCA type IV, according to Harding's classification.

In the present invention, the term "cerebellar ataxia in SCD" refers to ataxia caused by the damage of the cerebellum due to SCD and includes, for example, gait disturbance and stance disturbance (unsteady movement) in cerebellar ataxia.

The pharmaceutical compositions employed in the present invention exhibit effects in improving ataxia in SCD, and preferably, exhibit excellent effects in improving cerebellar ataxia in SCD. In one embodiment, a pharmaceutical composition exhibits excellent effects in improving gait and stance disturbances in ataxia of SCD, and preferably, exhibits excellent effects in improving one or more disorders selected from the group consisting of gait and stance disturbances in ataxia of SCD.

Effects of the pharmaceutical compositions in improving ataxia in SCD can be evaluated using, for example, SARA (Scale for the assessment and rating of ataxia) composed of eight test items (i.e., gait, stance, sitting, speech disturbance, finger chase, nose-finger test, fast alternating hand movements, and heel-shin slide) (see, for example, Neurology 2006, 66(11), p.1717-1720). Alternatively, by analyzing an effect in improving each of "gait" and "stance," the test items of SARA, the effects in improving gait and stance disturbances, which are important for the treatment of SCD, can be evaluated.

The dosage of the active ingredient is appropriately determined depending on, for example, the age, sex, body weight, extent of disease, genetic background, and/or the occurrence of side effects of the patient. The daily dose for adults can be determined in a range of 1.6 mg to 3.2 mg (calculated as a free form) for oral administration. For example, for adults, with an initial dose of 2.4 mg (calculated as a free form) of rovatirelin trihydrate, 2.4 mg (calculated as a free form) of rovatirelin trihydrate can be orally administered, which can be increased or decreased appropriately to the range of 1.6 mg to 3.2 mg of rovatirelin trihydrate based on a physician's judgment during the treatment period.

In one instance, when the initial dose is 1.6 mg (calculated as a free form), the daily dose for adults can be increased appropriately to 2.4 mg or 3.2 mg (calculated as a free form) or can be determined in the range of 1.6 mg to 3.2 mg (calculated as a free form), depending on the conditions, the presence or absence of side effects, and the like.

With regard to dosage and administration of the pharmaceutical compositions, rovatirelin or a pharmacologically acceptable salt thereof can be administered at a once daily dose of 1.6 mg to 3.2 mg (calculated as a free form). For example, rovatirelin trihydrate can be orally administered at a once daily dose of 2.4 mg as being calculated as a free form, which can be increased or decreased appropriately to the range of 1.6 mg to 3.2 mg (calculated as a free form) of rovatirelin trihydrate.

In one instance, for example, rovatirelin trihydrate can be orally administered at a once daily dose of 3.2 mg as being calculated as a free form, which can be decreased appropriately to the range of 1.6 mg to 2.4 mg (calculated as a free form) of rovatirelin trihydrate.

Furthermore, in one instance, for example, rovatirelin trihydrate can be orally administered at a once daily dose of 1.6 mg as being calculated as a free form, which can be increased appropriately to the range of 2.4 mg to 3.2 mg (calculated as a free form) of rovatirelin trihydrate.

In the present invention, "side effects caused by elevation of thyroid hormone levels" include direct or indirect side effects caused by elevations above the reference limit in levels of thyroid hormones (triiodothyronine (T₃) and thyroxine (T₄)). Specific events associated with side effects caused by elevation of thyroid hormone levels are, for example, an increase in blood pressure, an increase in heart rate, and a weight loss. In general, the risk of side effects caused by elevation of thyroid hormone levels can be estimated by measuring serum concentrations of thyroid stimulating hormone (TSH), free T₃ (FT₃), free T₄ (FT₄) or the like, although responsiveness varies from patient to patient. Typically, a reference range for FT₃ is 2.3-4.3 pg/mL and a reference range for FT₄ is 0.9-1.7 ng/dL. In order to reduce the risk of side effects caused by elevation of thyroid hormone levels, it is preferable that the FT₃ and FT₄ are not consistently above their reference ranges.

### EXAMPLES

Hereinafter, the present invention is described in more detail based on examples, but the present invention is not limited to the contents thereof. Each dosage (dose) of rovatirelin trihydrate in the examples is expressed by being calculated as its free form unless otherwise specified. Healthy adult males and SCD patients in the examples are Japanese unless otherwise specified and do not include those who exhibit ataxia secondarily (e.g., patients with cerebrovascular disorders, brain tumor and other diseases).

### EXAMPLE 1

### Repeated dose study in healthy adult males

### 1. Methods

To 50 healthy adult males (8 individuals per group and 10 on placebo), rovatirelin trihydrate (0.25, 0.5 or 1.0 mg) or placebo was orally administered once daily or rovatirelin trihydrate (0.25 or 0.5 mg) or placebo was orally administered twice daily after breakfast and after dinner, for 9 consecutive days.

### 2. Evaluation items

Serum concentrations (mean) of FT₃, FT₄, TSH, and prolactin (PRL), and adverse events and the like were evaluated.

### 3. Results

Figs. 1 and 2 show the transitions of serum concentrations (mean) of FT₃ and FT₄, respectively, on Day 1, Day 5, and Day 9 in each group.

When comparing the transition of FT₃ value of the treatment group that received 0.25 mg twice daily (0.25 mg/bid) with that of the treatment group that received 0.5 mg once daily (0.5 mg/qd), the transition for the 0.5 mg/qd group proceeded at lower levels as a whole. When comparing the transition of FT₃ value of the treatment group that received 0.5 mg twice daily (0.5 mg/bid) with that of the treatment group that received 1 mg once daily (1 mg/qd), the transition for the 1 mg/qd group proceeded at lower levels as a whole.

When comparing the transition of FT₄ values between the treatment group that received 0.25 mg twice daily (0.25 mg/bid) and the treatment group that received 0.5 mg once daily (0.5 mg/qd), the transitions were comparable for both groups or the transition for the 0.5 mg/qd group proceeded at slightly lower levels. When comparing the transition of FT₄ values between the treatment group that received 0.5 mg twice daily (0.5 mg/bid) and the treatment group that received 1 mg once daily (1 mg/qd), the transition for the 1 mg/qd group proceeded at lower levels as a whole, and the treatment group that received 0.5 mg twice daily consistently displayed FT₄ concentrations that were above the reference limit on Day 5 and Day 9.

The above analyses showed that influences of elevations in the thyroid hormone level caused by the repeated dosing of rovatirelin trihydrate can be reduced with a once-daily regimen compared with a twice-daily regimen. Thus, it was indicated that the risk of incidence of side effects, which are caused by elevation of thyroid hormone levels due to repeated dosing of rovatirelin trihydrate, can be reduced by the once-daily regimen.

### EXAMPLE 2

### Pharmacokinetic study in humans

Rovatirelin trihydrate (0.1, 0.3, 1, 2.5, 5 or 10 mg) or placebo was administered to 48 healthy adult males (6 individuals per group and 12 on placebo) as a single dose on an empty stomach. Analysis of the pharmacokinetics of intact rovatirelin under this condition indicated linearity of Cₘₐₓ (maximum plasma concentration), AUC_{0-∞} (area under the plasma concentration curve vs. time curve from time 0 to infinity), and Ae₀₋₄₈ (cumulative amount excreted into urine from time 0-48 hours) of rovatirelin in a dosage range from 0.1-10 mg.

From the results of Examples 1 and 2, with regard to the regimen of rovatirelin or a pharmacologically acceptable salt thereof, it was supposed that the risk of side effects caused by elevation of thyroid hormone levels due to the repeated dosing can be more reduced with the once-daily regimen than with the twice-daily regimen.

### EXAMPLE 3

### Clinical trial of SCD patients (phase II study)

### 1. Methods

Rovatirelin trihydrate (0.4, 0.8, 1.6 or 3.2 mg) or placebo was orally administered to 225 SCD patients once daily after breakfast for 24 weeks (double-blind).

### 2. Efficacy and safety endpoints

The efficacy endpoint was the amount of change in the SARA total score calculated by, as an example, the SARA total score at the final assessment in the treatment period (the final observed value in the treatment period) minus that at the end of the pre-observation period as an example. The safety endpoints were, as an example, the occurrence of adverse events and side effects, physiological examinations (e.g., blood pressure and pulses), and endocrinologic examinations (e.g., FT₃ and FT4).

It is noted that the SARA total score was calculated by a sum of the SARA scores for each item ((i.e., gait (score 0-8), stance (score 0-6), sitting (score 0-4), speech disturbance (score 0-6), finger chase (score 0-4), nose-finger test (score 0-4), fast alternating hand movements (score 0-4), and heel-shin slide (score 0-4) (the score 0 is normal for all items)).

### 3. Results of analysis

### (1) Efficacy

In amount of change in the SARA total score (mean) for the pure cerebellar SCD patients (23-28 individuals per group; total 126 individuals) from which one patient who had an extreme outlier result is excluded, dose-dependent improvements were observed (Table 1), suggesting that rovatirelin trihydrate at 1.6 mg or more has excellent effects in improving ataxia compared with placebo.

**[Table 1]**

| | | | | | |
|---|---|---|---|---|---|
| **Administered group** | 0.4 mg | 0.8 mg | 1.6 mg | 3.2 mg | placebo |
| **amount of change in the SARA total score (score**) | -1.09 | -1.27 | -1.58 | -1.65 | -0.85 |

### (2) Safety

Comparison between active drug and placebo groups using Fisher's exact test indicated that the treatment group that received 3.2 mg of rovatirelin trihydrate significantly differed in the side effect rate (P = 0.002). The treatment group that received 3.2 mg of rovatirelin trihydrate consistently displayed FT₃ and FT₄ concentrations that were slightly above the upper reference limits. Discontinuation rates were 15.6% in the placebo group, 17.8% in the 1.6 mg group, and 28.9% in the 3.2 mg group.

Considering the transition of thyroid hormones and incidence of side effects, 3.2 mg was considered to be excessive as an initial (baseline) dose despite some effects in improving ataxia and to be the maximum dose clinically applicable during the treatment period because SCD patients need to take medication over a long period of time. Furthermore, rovatirelin trihydrate at 0.4-0.8 mg was indicated to be safe but to have weak effects in improving ataxia in SCD patients. From these results, the clinically recommended dose in SCD patients was considered to be 1.6 mg.

### EXAMPLE 4

### Clinical trial of pure cerebellar SCD patients (phase III study)

### 1. Methods

Rovatirelin trihydrate at a dose of 1.6 or 2.4 mg or placebo was orally administered to patients with pure cerebellar SCD (SCA6, SCA31 or cortical cerebellar atrophy (CCA)) with ataxia once daily after breakfast for 28 weeks (double-blind; 124 patients in the 1.6 mg group, 122 patients in the 2.4 mg group, and 123 patients in the placebo group).

To appropriately assess the efficacy of rovatirelin trihydrate on ataxia, this study was performed on pure cerebellar SCD patients with a SARA gait score between 2 and 6 and a SARA total score of ≥6.

### 2. Efficacy and safety endpoints

The same items as those listed in Example 3 were used as efficacy and safety endpoints. SCD is a disease that automatically becomes worse; therefore it is important in the treatment to retard the deterioration. Accordingly, patients with a decrease in their SARA score compared with the time in week 0 were defined as deteriorated patients, and deterioration rates (i.e., the number of deteriorated patients/total number of patients) in the SARA total score and SARA scores for each item were calculated.

### 3. Results

### (1) Efficacy

The 2.4 mg group showed improvements in the SARA total score compared with the pre-dose score (amount of change in the SARA total score; -1.22). In addition, in the 2.4 mg group, the analysis of the SARA scores for each item indicated that rovatirelin exhibited effects in improving particularly gait and stance disturbances (unsteady movements) (Fig. 3). These improvement effects were more remarkable in the elderly aged ≥65 years with reduced muscular strength and less responsive to a placebo (amount of changes in the SARA total, gait, and stance scores; - 1.39, -0.19, and -0.54, respectively). Furthermore, the deterioration rate of the SARA total score was 30.9% in the placebo group, whereas it was 23.8% for the 2.4 mg group.

Although the 1.6 mg group also showed improvements in the SARA total score compared with the pre-dose score (amount of change in the SARA total score; -0.75), no improvement was observed in the SARA total score compared with the placebo group. Furthermore, no improvement was observed in the SARA gait and stance scores for the 1.6 mg group compared with the placebo group (Fig. 3).

### (2) Safety

Each of the FT₃ and FT₄ concentrations reached the upper limit of the normal range in the 1.6 mg and 2.4 mg groups 4 weeks after the start of administration but not further increase was found subsequently (see, Figs. 4 and 5). Discontinuation rates were 4.9% in the placebo group, 16.0% in the 1.6 mg group, and 19.8% in the 2.4 mg group.

Considering the transition of thyroid hormone and incidence of side effects, no clinically problematic event was observed with 1.6 and 2.4 mg doses of rovatirelin trihydrate, indicating that these doses can be used for long-term medication.

As a result of the aforementioned phase III study, according to the transition of thyroid hormones and incidence of side effects, it was indicated for the first time that 2.4 mg of rovatirelin trihydrate can be safely taken over a long period of time and can maximize benefits of improvement in ataxia (particularly gait disturbance) in consideration of the risk of side effects.

On the other hand, although 1.6 mg of rovatirelin trihydrate was found to be safe and some patients in the 1.6 mg group had a sufficient effect in improving ataxia, no sufficient improvement effect was unexpectedly found in improving gait and stance disturbances.

### EXAMPLE 5

### Long-term extension study of pure cerebellar SCD patients

### 1. Methods

For patients who completed the phase III study (Example 4), rovatirelin trihydrate was administered at 1.6 or 2.4 mg once daily for 52 weeks (open-label parallel-group comparative study).

### 2. Efficacy and safety endpoints

The same items as those listed in Example 4 were used as efficacy and safety endpoints, and deterioration rates (i.e., the number of deteriorated patients/total number of patients) in the SARA total score and SARA scores for each items were calculated in a similar manner.

### 3. Results of analysis

Improvement effects in ataxia were analyzed on patients in the 2.4 mg group in Example 4 (phase III study) continuing on rovatirelin trihydrate at 2.4 mg (administration period: a total of 52-week). The amount of change in the SARA total score was -1.41 at 52 weeks in the 81 patients who completed the 52-week administration, indicating a long-term effect of the 2.4 mg dose of rovatirelin trihydrate in improving ataxia. In addition, the deterioration rate of the SARA total score at 52 weeks in the same group was 21.0% and that for items (gait) at 52 weeks was 9.9%.

Although SCD is an intractable disease that automatically becomes worse, the deterioration rate of the SARA total score at 52 weeks was only 21.0% in patients who received rovatirelin continuously for a long period, suggesting that the administration of rovatirelin can delay the deterioration of ataxia in SCD patients.

### EXAMPLE 6

### Trials of SCD patients (study of switch from conventional drugs to rovatirelin)

### 1. Methods

Among SCD patients who had participated in a clinical trial of SCD patients using rovatirelin, those who had a SARA gait score between 2 and 6 and a SARA total score of ≥6 were the subjects in this study. To the patients were orally administered rovatirelin trihydrate at 1.6 or 2.4 mg once daily after breakfast for 24 weeks (randomized, open-label parallel-group comparative study).

A 4-week pre-observation period was set prior to the start of the treatment period. For patients who received a conventional SCD agent taltirelin or protirelin agent (TRH agent) before the pre-observation period, the medication was continued until the end of the pre-observation period without changing its dosage and administration.

### 2. Efficacy and safety endpoints

The same items as those listed in Example 4 were used as efficacy and safety endpoints.

### 3. Results of analysis

The efficacy of rovatirelin trihydrate was analyzed in pure cerebellar SCD patients who had taken taltirelin since before the start of the pre-observation period (switch group). Improvement effects of rovatirelin trihydrate were observed after 4 weeks of administration. A significant improvement in the SARA total score was observed for the switch group to which rovatirelin trihydrate was administered at 1.6 or 2.4 mg compared with the scores prior to the administration of rovatirelin trihydrate (treatment period with taltirelin) [amount of change in the SARA total score at an endpoint for the switch group that received 1.6 mg = -1.34 (P < 0.01), and that for the switch group that received 2.4 mg = -1.35 (P < 0.01)] (Fig. 6).

SCD is designated as an incurable disease of the nervous system and muscles. Since TRH formulations and taltirelin were the only agents for the treatment of SCD, a new therapeutic agent for SCD was desired. Example 6 suggests that rovatirelin has a greater effect than taltirelin as a therapeutic agent for improving ataxia in SCD.

No pharmaceutical agent was found to be effective in improving ataxia in SCD in clinical trials that used SARA which is a scale for the assessment of ataxia. Under such circumstances, rovatirelin was found to be effective in improving ataxia in SCD according to the SARA scales.

### Industrial Applicability

The pharmaceutical compositions employed in the present invention are particularly useful as therapeutic agents for ataxia in SCD.

## Claims

1. A pharmaceutical composition for use in the treatment of ataxia in spinocerebellar degeneration comprising, as an active ingredient, a daily dose of 1.6 mg to 3.2 mg of rovatirelin or 1.6 mg to 3.2 mg of pharmacologically acceptable salt of rovatirelin as being calculated as a free form, wherein the pharmaceutical composition is administered once daily.

2. The pharmaceutical composition according to Claim 1 for use according to claim 1, wherein 1.6 mg to 3.2 mg of rovatirelin trihydrate as being calculated as a free form is comprised as the active ingredient.

3. The pharmaceutical composition according to Claim 1 for use according to claim 1, wherein a daily dose of 2.4 mg of rovatirelin or 2.4 mg of pharmacologically acceptable salt of rovatirelin as being calculated as a free form is comprised as the active ingredient.

4. The pharmaceutical composition according to Claim 3 for use according to claim 3, wherein 2.4 mg of rovatirelin trihydrate as being calculated as a free form is comprised as the active ingredient.

5. The pharmaceutical composition according to any one of Claims 1 to 4 for use according to any one of Claims 1 to 4, wherein the ataxia in spinocerebellar degeneration is cerebellar ataxia in spinocerebellar degeneration.

6. The pharmaceutical composition according to any one of Claims 1 to 5 for use according to any one of Claims 1 to 5, wherein the pharmaceutical composition is an oral agent comprising rovatirelin or a pharmacologically acceptable salt thereof, and at least one pharmaceutical additive.

7. A pharmaceutical composition for use in the treatment of ataxia in spinocerebellar degeneration comprising, as an active ingredient, a daily dose of 1.6 mg, 2.4 mg, or 3.2 mg of rovatirelin or 1.6 mg, 2.4 mg, or 3.2 mg of pharmacologically acceptable salt of rovatirelin as being calculated as a free form in a single formulation.

## Patentansprüche

1. Pharmazeutische Zusammensetzung zur Verwendung bei der Behandlung von Ataxie bei spinozerebellärer Degeneration, umfassend als Wirkstoff eine tägliche Dosis von 1,6 mg bis 3,2 mg Rovatirelin oder 1,6 mg bis 3,2 mg eines pharmakologisch annehmbaren Salzes von Rovatirelin, berechnet als freie Form, wobei die pharmazeutische Zusammensetzung einmal täglich verabreicht wird.

2. Pharmazeutische Zusammensetzung gemäß Anspruch 1 zur Verwendung gemäß Anspruch 1, wobei 1,6 mg bis 3,2 mg Rovatirelin-Trihydrat, berechnet als freie Form, als Wirkstoff enthalten ist.

3. Pharmazeutische Zusammensetzung gemäß Anspruch 1 zur Verwendung gemäß Anspruch 1, wobei eine tägliche Dosis von 2,4 mg Rovatirelin oder 2,4 mg eines pharmakologisch annehmbaren Salzes von Rovatirelin, berechnet als freie Form, als Wirkstoff enthalten ist.

4. Pharmazeutische Zusammensetzung gemäß Anspruch 3 zur Verwendung gemäß Anspruch 3, wobei 2,4 mg Rovatirelin-Trihydrat, berechnet als freie Form, als Wirkstoff enthalten ist.

5. Pharmazeutische Zusammensetzung gemäß einem der Ansprüche 1 bis 4 zur Verwendung gemäß einem der Ansprüche 1 bis 4, wobei die Ataxie bei spinozerebellärer Degeneration eine zerebelläre Ataxie bei spinozerebellärer Degeneration ist.

6. Pharmazeutische Zusammensetzung gemäß einem der Ansprüche 1 bis 5 zur Verwendung gemäß einem der Ansprüche 1 bis 5, wobei die pharmazeutische Zusammensetzung ein orales Mittel ist, das Rovatirelin oder ein pharmakologisch annehmbares Salz davon und mindestens einen pharmazeutischen Zusatzstoff umfasst.

7. Pharmazeutische Zusammensetzung zur Verwendung bei der Behandlung von Ataxie bei spinozerebellärer Degeneration, umfassend als Wirkstoff eine tägliche Dosis von 1,6 mg, 2,4 mg oder 3,2 mg Rovatirelin oder 1,6 mg, 2,4 mg oder 3,2 mg eines pharmakologisch annehmbaren Salzes von Rovatirelin, berechnet als freie Form, in einer einzigen Formulierung.

## Revendications

1. Composition pharmaceutique à utiliser pour le traitement de l'ataxie associée à la dégénérescence spino-cérébelleuse comprenant, comme principe actif, une dose journalière de 1,6 mg à 3,2 mg de rovatiréline ou de 1,6 mg à 3,2 mg de sel pharmacologiquement acceptable de rovatiréline calculée sous forme libre, dans laquelle la composition pharmaceutique est administrée une fois par jour.

2. Composition pharmaceutique selon la revendication 1 à utiliser selon la revendication 1, dans laquelle de 1,6 mg à 3,2 mg de trihydrate de rovatiréline calculés sous forme libre sont compris comme principe actif.

3. Composition pharmaceutique selon la revendication 1 à utiliser selon la revendication 1, dans laquelle une dose journalière de 2,4 mg de rovatiréline ou de 2,4 mg de sel pharmacologiquement acceptable de rovatiréline calculée sous forme libre est comprise comme principe actif.

4. Composition pharmaceutique selon la revendication 3 à utiliser selon la revendication 3, dans laquelle 2,4 mg de trihydrate de rovatiréline calculés sous forme libre sont compris comme principe actif.

5. Composition pharmaceutique selon l'une quelconque des revendications 1 à 4 à utiliser selon l'une quelconque des revendications 1 à 4, dans laquelle l'ataxie associée à la dégénérescence spino-cérébelleuse est une ataxie cérébelleuse associée à la dégénérescence spino-cérébelleuse.

6. Composition pharmaceutique selon l'une quelconque des revendications 1 à 5 à utilise selon l'une quelconque des revendications 1 à 5, dans laquelle la composition pharmaceutique est un agent oral comprenant de la rovatiréline ou un sel pharmacologiquement acceptable de celle-ci, et au moins un additif pharmaceutique.

7. Composition pharmaceutique à utiliser pour le traitement de l'ataxie associée à la dégénérescence spino-cérébelleuse comprenant, comme principe actif, une dose journalière de 1,6 mg, 2,4 mg ou 3,2 mg de rovatiréline ou de 1,6 mg, 2,4 mg ou 3,2 mg de sel pharmacologiquement acceptable de rovatiréline calculée sous forme libre dans une formulation unique.
